# EUROPEAN PATENT APPLICATION

(11) **EP 3 789 085 A1**
(43) Date of publication of application: **10.03.2021**
(21) Application number: 19290087.6
(22) Date of filing: 05.09.2019
(51) Int. Cl.: A61N 5/10

(54) **DOSE-GUIDED DEFORMABLE IMAGE REGISTRATION**

(71) Applicant: Koninklijke Philips N.V., 5656 AG Eindhoven (NL)
(72) Inventor: Allaire, Stéphane, 5656 AE Eindhoven (NL)
(74) Representative: de Haan, Poul Erik

(57) **Abstract**

The present invention relates to radiation therapy. In order to improve the accuracy in deformable image registration in radiation therapy planning, a method is provided that comprises a) receiving a dose distribution to be delivered during one or more treatment sessions according to a radiation therapy plan, b) receiving contours delineating at least one region of interest, ROI, of at least one of the medical images, c) receiving one or more treatment objectives associated with the at least one ROI that has delineated contours, d) determining one or more critical regions in at least one of the medical images, where a geometric error of the contours of the at least one ROI and/or an uncertainty in the electron density distribution leads to a violation of one or more treatment objectives with respect to the dose distribution, and e) improving the accuracy of a deformable image registration algorithm in the one or more critical regions for registering the at least two medical images, wherein the deformable image registration algorithm estimates a deformation between the at least two medical images.

## Description

### FIELD OF THE INVENTION

The present invention relates to radiation therapy. In particular, the present invention relates to a radiation therapy planning method and a radiation therapy planning apparatus. The present invention further relates to a computer program element.

### BACKGROUND OF THE INVENTION

Deformable image registration (DIR) is key in radiation therapy, adaptive planning, composite planning, with accuracy requirements intensified by the hypofractionation trend.

In radiation therapy, DIR is used to estimate deformation between images used for treatment planning and/or guidance, and thereafter to map radiation therapy planning region-of-interest contours (ROIs), map computer tomography (CT) densities onto another modality, such as cone beam CT (CBCT) in adaptive RT, magnetic resonance (MR) in MR-only simulation (MRCAT), to create a virtual CT or attenuation map, and accumulate dose. The radiation therapy planning ROIs are subject to dose objective constraints in the planning.

Although various DIR approaches have been proposed for the RT, DIR geometric errors may occur, which may have a great impact on dosimetric errors.

### SUMMARY OF THE INVENTION

There may be a need to improve the DIR accuracy in radiation therapy planning.
The object of the present invention is solved by the subject-matter of the independent claims, wherein further embodiments are incorporated in the dependent claims. It should be noted that the following described aspects of the invention apply also for the radiation therapy planning method, the radiation therapy planning apparatus, and the computer program element.

A first aspect of the present invention provides a radiation therapy planning method for registering at least two three-dimensional medical images of a patient, comprising the following steps:
a) receiving a dose distribution to be delivered during one or more treatment sessions according to a radiation therapy plan;
b) receiving contours delineating at least one region of interest, ROI, of at least one of the medical images;
c) receiving one or more treatment objectives associated with the at least one ROI that has delineated contours;
d) determining one or more critical regions in at least one of the medical images, where a geometric error of the contours of the at least one ROI and/or an uncertainty in the electron density distribution leads to a violation of one or more treatment objectives with respect to the dose distribution; and
e) improving the accuracy of a deformable image registration algorithm in the one or more critical regions for registering the at least two medical images, wherein the deformable image registration algorithm estimates a deformation between the at least two medical images.

A further aspect of the present invention provides a radiation therapy planning apparatus with a configuration for registering at least two three-dimensional image sets of a patient. The radiation therapy planning apparatus comprises:
- an input unit; and
- a processing unit;
wherein the input unit is configured to receive a dose distribution to be delivered during one or more treatment sessions according to a radiation therapy plan, to receive contours delineating at least one region of interest, ROI, of at least one of the medical images, to receive one or more treatment objectives associated with the at least one ROI that has delineated contours; and
wherein the processing unit is configured to determine one or more critical regions in at least one of the medical images, where a geometric error of the contours of the at least one ROI and/or an uncertainty in the electron density distribution leads to a violation of one or more treatment objectives with respect to the dose distribution, and to improve the accuracy of a deformable image registration algorithm in the one or more critical regions for registering the at least two medical images, wherein the deformable image registration algorithm estimates a deformation between the at least two medical images.

A further aspect of the present invention provides a computer program element, which, when being executed by at least one processing unit, is adapted to cause the processing unit to perform the method.

DIR may be seen as a whole that encompasses two phases: a) estimation of the deformation between two or more three-dimensional medical images of a patient, and b) application of that deformation to any object. For the estimation of the deformation, i.e. phase a), at least two three-dimensional medical images of the patient are entered into the DIR for generating a deformation map, such as a displacement vector field (DVF). For the application of the deformation, i.e. phase b), the deformation map is applied to warp over any image content (e.g., pixel intensities), any region of interest ROI (e.g., represented as contours, mesh, mask, etc.), any point of interest (POI) (e.g., landmark), any dose grid, etc., of one medical image into alignment with the other respective medical image.

In the present disclosure, a dose-guided DIR is proposed. In other words, it is proposed to use a DIR approach with locally improved accuracy in the regions where some clues indicate that DIR accuracy matters in terms of radiation therapy dosimetry and treatment goals. For example, the DIR accuracy may matter in the regions where contour mapping accuracy matters, e.g., for parotid gland contours in HN cancer patient cases in long range dose variations, or for spinal cord close to high dose regions or dose gradient. For example, the DIR accuracy may matter in the regions where density mapping accuracy matters for a virtual attenuation map, e.g., at the patient outline, the bone structures, and internal air cavities.

The proposed dose-guided DIR requires for the estimation of the deformation, i.e. phase a) two extra inputs: a dose distribution and one or more treatment objectives, i.e. ROI dose constraints, associated with each ROI that has delineated contours. With these two extra inputs, it is analysed where a geometric error of the contours of the at least one ROI and/or an uncertainty in the electron density distribution leads to a violation of one or more treatment objectives with respect to the dose distribution. There regions are named as critical regions. Critical regions may include critical spots, i.e. points in the medical image where geometric errors and/or uncertainty in the electron density distribution at these points can violate the treatment objective with respect to the dose distribution. Critical regions may include critical slices, i.e., image slices where geometric errors and/or uncertainty in the electron density distribution in these image slices can violate the treatment objective with respect to the dose distribution. The term "violate" refers to the situation where the treatment objective is not met. Once these critical regions are determined, the local accuracy of the DIR in these critical regions can be boosted or improved. Several DIR approaches have the ability to include local refinement to improve the local accuracy in these critical regions. For example, with B-splines it is possible to define a locally finer grid, e.g., local refinement of hierarchical B-splines. For example, Sparse Demons can directly drive local refinement due to the sparsity accounted for in the variational equations and implementations. Another example of the DIR approach is Salient-Feature-Based-Registration (SFBR), which flexibly enables locally boosted DIR, through the possibility of regional settings for feature extraction and/or feature matching. By improving local accuracy in these critical regions, the critical impactful DIR error can be avoided in the first place. The dose-guided DIR may also allow to speed up DIR without making too many concessions on the quality-speed trade-off. These critical regions, e.g., critical spots and/or critical slices, may be presented to a user to guide and accelerate contour review by the user.

Optionally, the deformation map may be generated by the dose-guided DIR for phase b). The deformation map may be used for deforming, i.e. propagating non-rigidly, any object, such as contours, landmarks, pixel intensities, into alignment with the respect image set. The radiation therapy plan can also be updated accordingly. A dose accumulation may be performed based on the updated radiation therapy plan.

According to an embodiment of the present invention, the method further comprises the following steps: determining one or more critical spots in at least one of the medical images where a geometric error and/or an uncertainty in the electron density distribution at the one or more spots leads to a violation of one or more treatment objectives with respect to the dose distribution, and/or determining one or more critical slices in at least one of the medical images, where a geometric error and/or an uncertainty in the electron density distribution at the one or more spots leads to a violation of one or more treatment objectives with respect to the dose distribution.

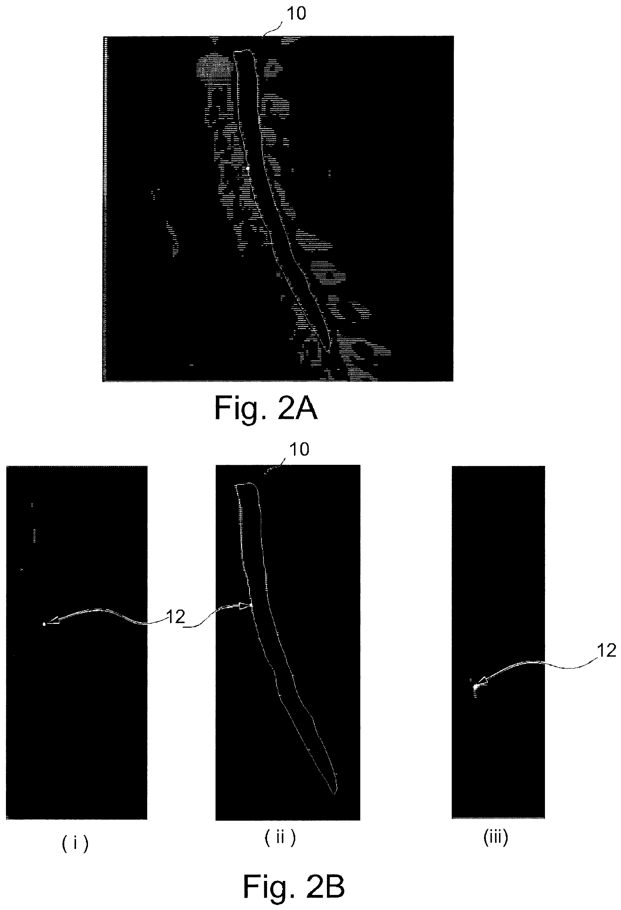

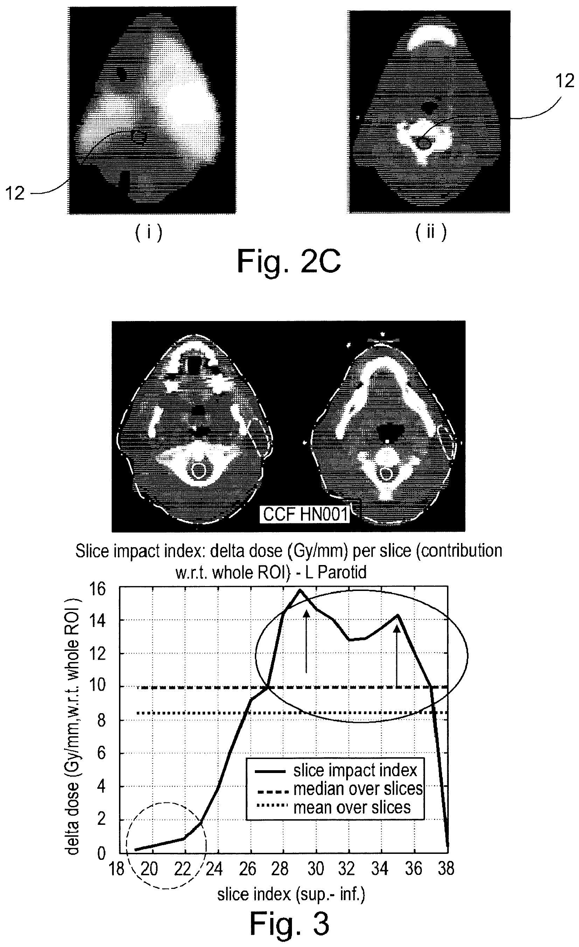

Critical spots refer to the spots where most critical contour point of the ROI is located therein, with respect to the nearby dose distribution and the treatment objectives. The critical spots are not necessarily towards visually obvious highest target doses or dose gradients. Critical slices may be determined by calculating a slice impact index in each image slice and selecting the image slices with highest slice impact index, or at least one with local maxima. The critical spots and the critical slices may be presented on a display to guide and accelerate contour review by the user. Multivalued masks representation may be used to present the critical spots and the critical slices. The critical spots and the critical slices may also be presented by color-coded risk along the contours, or by being displayed in CT image with contours and isodose lines. This will be explained in detail with respect to Fig. 2 and Fig. 3.

According to an embodiment of the present invention, the one or more treatment objectives comprise at least one of a minimum/maximum value of a dose, a minimum/maximum value of an average dose, and a minimum/maximum value of a dose-volume histogram curve point.

According to an embodiment of the present invention, the one or more critical regions in at least one of the medical images are determined where a geometric error of the contours of the at least one ROI and/or an uncertainty in the electron density distribution leads to a violation of one or more treatment objectives with respect to the dose distribution up to a given distance from the at least one ROI.

As the contour mapping accuracy or the density mapping accuracy may matter in the regions close to the ROI, the consideration of the critical regions close to the ROI may further improve the accuracy of the dose-guided DIR.

According to an embodiment of the present invention, the deformable image registration algorithm is configured to perform deformable image registration with locally improved accuracy based on the following approaches: B-splines, Sparse Demons, and/or Salient-Feature-Based Registration (SFBR).

For example, with B-splines it is possible to define a locally finer grid, e.g., local refinement of hierarchical B-splines. For example, Sparse Demons can directly drive local refinement due to the sparsity accounted for in the variational equations and implementations. Another example of the DIR approach is Salient-Feature-Based-Registration (SFBR), which flexibly enables locally boosted DIR, through the possibility of regional settings for feature extraction and/or feature matching.

According to an embodiment of the present invention, the method further comprises the step of generating a deformation map, which is a non-rigid transformation that maps the at least two three-dimensional image sets. Preferably, the generated deformation map is provided as a displacement vector field.

The deformation map generated from the dose-guided DIR has a better accuracy in the critical regions, where DIR accuracy matters in terms of dosimetry and treatment goals.

According to an embodiment of the present invention, the method further comprises applying the generated deformation map to warp over at least one of an image content, a ROI, a point of interest, POI, and a dose grid of the one medical image into alignment with the other respective medical image.

As the deformation has a better accuracy in the critical regions, the warping result is more accurate at least in the critical regions.

According to an embodiment of the present invention, the method further comprises updating the radiation therapy plan based on the at least two registered medical images.

According to an embodiment of the present invention, the method further comprises performing a dose accumulation based on the updated radiation therapy plan.

According to an embodiment of the present invention, the processing unit is configured to determine one or more critical spots in at least one of the medical images where a geometric error and/or an uncertainty in the electron density distribution at the one or more spots leads to a violation of one or more treatment objectives with respect to the dose distribution, and/or to determining one or more critical slices in at least one of the medical images, where a geometric error and/or an uncertainty in the electron density distribution at the one or more spots leads to a violation of one or more treatment objectives with respect to the dose distribution.

According to an embodiment of the present invention, the apparatus comprises a display for displaying the one or more critical spots and/or the one or more critical slices.

According to an embodiment of the present invention, the one or more treatment objectives comprise at least one of a minimum/maximum value of a dose, a minimum/maximum value of an average dose, and a minimum/maximum value of a dose-volume histogram curve point.

According to an embodiment of the present invention, the processing unit is configured to register the at least two three-dimensional medical images and to generate a deformation map or displacement vector field that can then be applied to warp over at least one of an image content, a ROI, a point of interest, POI, and a dose grid of one medical image into alignment with the other respective medical image.

Although the present disclosure is discussed with respect to the intra-patient DIR use case, a person skilled in the art will appreciate that the apparatus and computer-implemented method may be used for other applications. For example, the radiation therapy planning ROI contours may be mapped to adaptive radiation therapy (ART), e.g., from planning over to re-planning CT images, or from planning over to CBCT. In another example, the dose-driven principle may be applied in MR-only simulation to generate MRCAT images (magnetic resonance for calculating attenuation). In a further example, the dose-driven principle may be applied for atlas-based auto-segmentation through DIR. In another example, CT densities may be mapped onto a same patient 3D image from another imaging modality to create a virtual CT from which is derived the electron density distribution, which is used as an attenuation map to estimate/predict the deposited dose distribution, for example, CBCT in adaptive image-guided radiation therapy (IGRT), with accuracy requirements intensified by the hypofractionation trend nowadays where a higher dose is delivered in each of the fewer treatment fractions, or MR in in MR-only simulation to generate MRCAT images. Another application is to map and accumulate dose across time points through one treatment, or across treatments in composite planning, i.e., when planning a new radiation therapy treatment, e.g., to address cancer recurrence.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 shows an example of a radiation therapy planning method for registering at least two three-dimensional medical images of a patient.
Fig. 2A shows an example of a contour 10 delineating a spinal cord of a patient.
Fig. 2B shows an example of a ROI-specific local criticalness map (i) in a sagittal view and (ii) in an axial view.
Fig. 2C shows an example of a critical spot (i) in a dose distribution map and (ii) in auto-presented CT slice.
Fig. 3C shows an example of critical slices.
Fig. 4 shows an example of a radiation therapy planning apparatus.

It should be noted that the figures are purely diagrammatic and not drawn to scale. In the figures, elements which correspond to elements already described may have the same reference numerals. Examples, embodiments, and optional features, whether indicated as non-limiting or not, are not to be understood as limiting the invention as claimed.

### DETAILED DESCRIPTION OF EMBODIMENTS

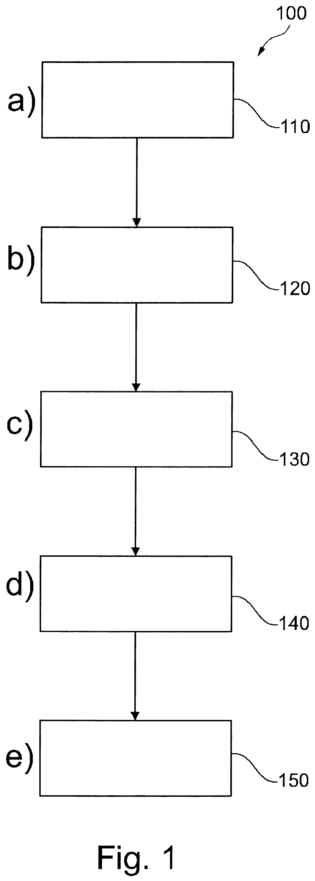

Fig. 1 shows a radiation therapy planning method 100 for registering at least two three-dimensional medical images of a patient. The method comprises the following steps:
In step 110, i.e. step a), a dose distribution to be delivered during one or more treatment sessions according to a radiation therapy plan is received.

In step 120, i.e. step b), contours delineating at least one region of interest, ROI, of at least one of the medical images are received. Automatic segmentation tools may be used to delineate the at least one ROI. Alternatively or additionally, manual segmentation tools may be used which allow a user to manually adjust image features, particularly the segmentation contours and/or the landmark positions when the medical images are displayed on a display. As an example, Fig. 2A illustrates a contour 10 delineating a spinal cord, i.e. an example of the ROI, of a patient.

In step 130, i.e. step c), one or more treatment objectives associated with the at least one ROI that has delineated contours are received. The one or more treatment objectives, which describe desired spatially varying treatment objectives to be applied to a target region, are typically entered or confirmed by a clinician or other human user using a user interface, such as a computer-implemented graphical user interface (GUI) or other suitable operator interface. The one or more treatment objectives may include one or more of a minimum radiation dose to be applied in the target region, a maximum dose to be applied to a region outside the target (e.g., to an organ at risk or otherwise healthy tissue), minimum and maximum dose-volume objectives, dose uniformity objectives, and the like.

In step 140, i.e. step d), one or more critical regions are determined in at least one of the medical images, where a geometric error of the contours of the at least one ROI and/or an uncertainty in the electron density distribution leads to a violation of one or more treatment objectives with respect to the dose distribution. In other words, the impact of geometric errors of the DIR and/or the uncertainty in the electron density distribution are analyzed onto dosimetric mis-estimation in radiation therapy planning.

For example, CBCT images have the potential to delineate the patient anatomy online and determine the tissues' electron densities which can be used to calculate the dose for adaptive treatment planning. The conversion of the CT number (in Hounsfield units, HU) into an electron density enables the treatment planning system to account for tissue heterogeneities. Therefore, the accuracy of patient dose calculations is largely dependent on the accuracy of the electron density. One limitation of CBCT image quality is the serious artifacts introduced by X-ray scatter and beam hardening. This will reduce contrast resolution and increase noise in CBCT images, therefore affecting the HU and electron density values. Alternatively patient dose calculation may be performed on a DIR-based virtual CT image that may have been produced by registering and warping some real CT image onto the CBCT image. Either way, the uncertainty in the electron density distribution may lead to a violation of one or more treatment objectives with respect to the dose distribution.

Examples of the critical regions may include critical spots and critical slices. An example of critical spots is illustrated in Figs. 2B and 2C, and an example of the critical slices is illustrated in Fig. 3.

Critical spots are the spots in at least one of the medical images, where a geometric error and/or an uncertainty in the electron density distribution at the one or more critical spots leads to a violation of one or more treatment objectives with respect to the dose distribution. For example, Fig. 2B shows a ROI-specific local criticalness map (i) in a sagittal view, (ii) in a sagittal view with superimposed entire spinal cord contour 10, and (iii) in an axial view. It is noted that the bright spots of the local criticalness map in Fig. 2B(i) are a subset of the points of the spinal cord contour 10 as illustrated in Fig. 2B(ii). This shows that only the corresponding slices would need to be reviewed in priority by a clinical expert, which can potentially accelerate the contour review process. The ROI-specific local criticalness map shows a critical spot 12, i.e. top risk spot, where geometric errors and/or an uncertainty in the electron density distribution can violate the treatment objective with respect to the dose distribution. In the example of Fig. 2B (iii), the local criticalness map is displayed in the critical slice which includes the most critical spot 12 which was determined among the spinal cord contour. In the example of Fig. 2B, the overall local criticalness map was computed up to a given distance of 15mm from the spinal cord ROI. Optionally, if the spot with highest risk, i.e. spot where most critical contour point of the ROI is located with respect to nearby dose distribution, is found, the method may further comprise automatically presenting the image slice where the most dose-critical contour point is located. For example, Fig. 2C shows the critical spot 12 (i) in a dose distribution map and (ii) in an auto-presented CT slice. In other words, this is where the most critical contour point of the ROI is located with respect to the nearby dose distribution in the dose distribution map. The CT slice may be presented to a user to guide and accelerate contour review by the user.

Another example of the critical regions may include one or more critical slices in at least one of the medical images, where a geometric error and/or an uncertainty in the electron density distribution in the one or more critical slices leads to a violation of one or more treatment objectives with respect to the dose distribution. In Fig. 3, for example, a slice impact index may be calculated for a plurality of image slices of the ipsilateral parotid ROI. In the example of Fig. 3, the slice impact index has two local maxima. The corresponding CT slices may be considered as top-priority slices that require a user to review the ROI auto-mapped contour, or to drive local DIR refinement beforehand.

In step 150, i.e. step e), the accuracy of a deformable image registration algorithm in the one or more critical regions for registering the at least two medical images is improved. The deformable image registration algorithm estimates a deformation between the at least two medical images. In other words, the deformation estimation or the accuracy of the DIR algorithm is locally improved or boosted. In particular, the accuracy of the DIR algorithm is improved in the one or more critical regions. And then, the subsequent registration, e.g., warping, will be affected, possibly locally e.g., with B-splines, or globally, if e.g. the DIR algorithm uses a parametric transform with global domain like thin-plate splines.

Several DIR approaches have the ability to include local refinement. For example, in some intensity-based approaches like B-splines, it is possible to define a locally finer grid, e.g., local refinement of hierarchical B-splines. In another example, Sparse Demons may directly drive local refinement, by design thanks to the sparsity accounted for in the variational equation and implementation. A further approach may include Salient-Feature-Based-Registration (SFBR), which flexibly enables locally boosted DIR through the possibility of regional settings for feature extraction and/or feature matching.

It will be appreciated that the above operation may be performed in any suitable order, e.g., consecutively, simultaneously, or a combination thereof, subject to, where applicable, a particular order being necessitated, e.g., by input/output relations.

Optionally, the method may further comprise the step of generating a deformation map, which is a non-rigid transformation that maps the at least two three-dimensional image sets. The result of DIR is the deformation map, which describes the spatial correspondence between two or among multiple sets of images. Preferably, the generated deformation map is provided as a DVF, which may be displayed as arrows. The size and direction of the arrows represent the magnitude and direction of DVF.

The generated deformation map may be applied to warp over at least one of an image content, a ROI, a point of interest, POI, and a dose grid of the one medical image into alignment with the other respective medical image. In other words, it is possible to apply the deformation map to warp over any of the images, contours, or further received images, contours, landmarks, into alignment with the respective image set.

As a new or correct deformation map is generated, the radiation therapy plan may be updated based on the at least two registered medical images. A dose accumulation may also be updated based on the updated radiation therapy plan. To update the accumulated dose in a first treatment session, the correct deformation map may be applied to the subsequent plan to conform the ROIs and their dose to the planning image for a second treatment session. In this manner, the accumulated radiation in each ROI is accurately determined over a plurality of treatment sessions even as ROIs shift, shrink, expand, change shape or the like.

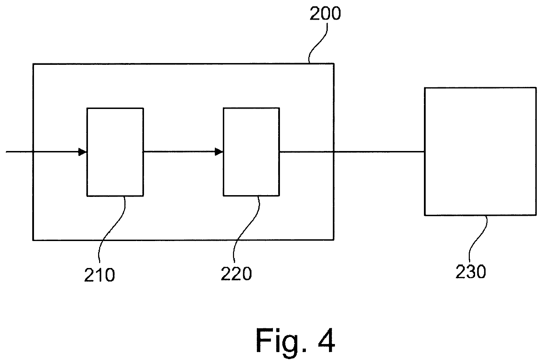

Fig. 4 schematically shows a radiation therapy planning apparatus 200 with a configuration for registering at least two three-dimensional image sets of a patient. The radiation therapy planning apparatus 200 comprises an input unit 210, and a processing unit 220.

The input unit 210 is configured to receive a dose distribution to be delivered during one or more treatment sessions according to a radiation therapy plan.

The input unit 210 is further configured to receive contours delineating at least one region of interest, ROI, of at least one of the medical images.

The input unit 210 is further configured to receive one or more treatment objectives associated with the at least one ROI that has delineated contours. The one or more treatment objectives comprise at least one of a minimum/maximum value of a dose, a minimum/maximum value of an average dose, and a minimum/maximum value of a dose-volume histogram curve point.

The processing unit 220 is configured to determine one or more critical regions in at least one of the medical images, where a geometric error of the contours of the at least one ROI and/or an uncertainty in the electron density distribution leads to a violation of one or more treatment objectives with respect to the dose distribution.

The processing unit 220 is configured to improve the accuracy of a deformable image registration algorithm in the one or more critical regions for registering the at least two medical images, wherein the deformable image registration algorithm estimates a deformation between the at least two medical images. In other words, the processing unit 220 is configured to boost the local accuracy of the DIR in the critical regions. Several approaches may be used to improve the local accuracy of the DIR, such as B-splines, Sparse Demons, and Salient-Feature-Based-Registration (SFBR).

Optionally, the processing unit is configured to determine one or more critical spots in at least one of the medical images where a geometric error and/or an uncertainty in the electron density distribution at the one or more spots leads to a violation of one or more treatment objectives with respect to the dose distribution, and/or to determining one or more critical slices in at least one of the medical images, where a geometric error and/or an uncertainty in the electron density distribution at the one or more spots leads to a violation of one or more treatment objectives with respect to the dose distribution.

Optionally, the apparatus comprises a display 230 for displaying the one or more critical spots and/or the one or more critical slices. Optionally, these critical spots and/or critical slices may be presented to a user to guide and accelerate contour review by the user.

Optionally, the processing unit 220 is configured to register the at least two three-dimensional medical images and to generate a deformation map or displacement vector field that can then be applied to warp over at least one of an image content, a ROI, a POI, and a dose grid of one medical image into alignment with the other respective medical image.

The radiation therapy planning apparatus 200 may be embodied as, or in, a device or apparatus, such as a server, workstation, imaging system or mobile device. The radiation therapy planning apparatus 200 may comprise one or more microprocessors or computer processors which execute appropriate software. The processor for the apparatus may be embodied by one or more of these processors. The software may have been downloaded and/or stored in a corresponding memory, e.g., a volatile memory such as RAM or a non- volatile memory such as Flash. The software may comprise instructions configuring the one or more processors to perform the functions described with reference to the processor of the apparatus. Alternatively, the functional units of the apparatus, e.g., the input unit, and the processing unit, may be implemented in the device or apparatus in the form of programmable logic, e.g., as a Field-Programmable Gate Array (FPGA). In general, each functional unit of the apparatus may be implemented in the form of a circuit. It is noted that the apparatus 200 may also be implemented in a distributed manner, e.g., involving different devices or apparatuses.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A radiation therapy planning method for registering at least two three-dimensional medical images of a patient, comprising the following steps:
a) receiving a dose distribution to be delivered during one or more treatment sessions according to a radiation therapy plan;
b) receiving contours delineating at least one region of interest, ROI, of at least one of the medical images;
c) receiving one or more treatment objectives associated with the at least one ROI that has delineated contours;
d) determining one or more critical regions in at least one of the medical images, where a geometric error of the contours of the at least one ROI and/or an uncertainty in the electron density distribution leads to a violation of one or more treatment objectives with respect to the dose distribution; and
e) improving the accuracy of a deformable image registration algorithm in the one or more critical regions for registering the at least two medical images, wherein the deformable image registration algorithm estimates a deformation between the at least two medical images.

2. Method according to claim 1, further comprising:
- determining one or more critical spots in at least one of the medical images where a geometric error and/or an uncertainty in the electron density distribution at the one or more spots leads to a violation of one or more treatment objectives with respect to the dose distribution; and/or
- determining one or more critical slices in at least one of the medical images, where a geometric error and/or an uncertainty in the electron density distribution in the one or more critical slices leads to a violation of one or more treatment objectives with respect to the dose distribution.

3. Method according to claim 1 or 2,
wherein the one or more treatment objectives comprise at least one of a minimum/maximum value of a dose, a minimum/maximum value of an average dose, and a minimum/maximum value of a dose-volume histogram curve point.

4. Method according to any one of the preceding claims,
wherein the one or more critical regions in at least one of the medical images are determined where a geometric error of the contours of the at least one ROI and/or an uncertainty in the electron density distribution leads to a violation of one or more treatment objectives with respect to the dose distribution up to a given distance from the at least one ROI.

5. Method according to any of the preceding claims,
wherein the deformable image registration algorithm is configured to perform deformable image registration with locally improved accuracy based on the following approaches:
- B-splines;
- Sparse Demons; and/or
- Salient-Feature-Based Registration, SFBR.

6. Method according to any of the preceding claims, further comprising:
- generating a deformation map, which is a non-rigid transformation that maps the at least two three-dimensional image sets;
wherein, preferably, the generated deformation map is provided as a displacement vector field.

7. Method according to claim 6, further comprising:
- applying the generated deformation map to warp over at least one of an image content, a ROI, a point of interest, POI, and a dose grid of the one medical image into alignment with the other respective medical image.

8. Method according to any one of the preceding claims, further comprising:
- updating the radiation therapy plan based on the at least two registered medical images.

9. Method according to claim 8, further comprising:
- performing a dose accumulation based on the updated radiation therapy plan.

10. A radiation therapy planning apparatus with a configuration for registering at least two three-dimensional image sets of a patient, comprising:
- an input unit; and
- a processing unit;
wherein the input unit is configured to receive a dose distribution to be delivered during one or more treatment sessions according to a radiation therapy plan, to receive contours delineating at least one region of interest, ROI, of at least one of the medical images, to receive one or more treatment objectives associated with the at least one ROI that has delineated contours; and
wherein the processing unit is configured to determine one or more critical regions in at least one of the medical images, where a geometric error of the contours of the at least one ROI and/or an uncertainty in the electron density distribution leads to a violation of one or more treatment objectives with respect to the dose distribution, and to improve the accuracy of a deformable image registration algorithm in the one or more critical regions for registering the at least two medical images, wherein the deformable image registration algorithm estimates a deformation between the at least two medical images.

11. Apparatus according to claim 10,
wherein the processing unit is configured to:
- determine one or more critical spots in at least one of the medical images where a geometric error and/or an uncertainty in the electron density distribution at the one or more spots leads to a violation of one or more treatment objectives with respect to the dose distribution; and/or
- to determining one or more critical slices in at least one of the medical images, where a geometric error and/or an uncertainty in the electron density distribution in the one or more image slices leads to a violation of one or more treatment objectives with respect to the dose distribution.

12. Apparatus according to any one of claims 10 or 11,
wherein the apparatus comprises a display for displaying the one or more critical spots and/or the one or more critical slices.

13. Apparatus according to any one of claims 10 to 12,
wherein the one or more treatment objectives comprise at least one of a minimum/maximum value of a dose, a minimum/maximum value of an average dose, and a minimum/maximum value of a dose-volume histogram curve point.

14. Apparatus according to claim 11 or 12,
wherein the processing unit is configured to register the at least two three-dimensional medical images and to generate a deformation map or displacement vector field that can then be applied to warp over at least one of an image content, a ROI, a point of interest, POI, and a dose grid of one medical image into alignment with the other respective medical image.

15. A computer program element, which, when being executed by at least one processing unit, is adapted to cause the processing unit to perform the method as per claims 1 to 9.
